# EUROPÄISCHE PATENTSCHRIFT

(11) **EP 3 634 525 B1**
(45) Veröffentlichungstag und Bekanntmachung des Hinweises auf die Patenterteilung: **12.07.2023**
(21) Anmeldenummer: 18729394.9
(22) Anmeldetag: 04.06.2018
(51) Int. Cl.: A61M 60/117, A61M 60/178, A61M 60/183, A61M 60/216, A61M 60/531, A61M 60/554, A61M 60/857, A61M 60/861

(54) **HERZUNTERSTÜTZUNGSSYSTEM**
HEART SUPPORT SYSTEM
DISPOSITIF D'ASSISTANCE VENTRICULAIRE

(30) Priorität: 06.06.2017 DE 102017112437
(43) Veröffentlichungstag der Anmeldung: 15.04.2020
(73) Patentinhaber: Rheinisch-Westfälische Technische Hochschule (RWTH) Aachen, 52062 Aachen (DE)
(72) Erfinder: GESENHUES, Jonas, 52064 Aachen (DE); ABEL, Dirk, 52072 Aachen (DE)
(74) Vertreter: Michalski Hüttermann & Partner Patentanwälte mbB
(86) Internationale Anmeldenummer: PCT/EP2018/064617
(87) Internationale Veröffentlichungsnummer: WO 2018/224436

(56) Entgegenhaltungen:
- US-A- 5 186 431
- US-A- 5 270 005
- US-A- 5 928 179
- US-A- 6 017 493
- US-A1- 2007 208 290
- US-A1- 2017 112 993

## Beschreibung

Die Erfindung betrifft ein Herzunterstützungssystem mit mindestens einer Systemeinheit, die eine Pumpenanordnung, eine an die Pumpenanordnung angeschlossene erste Anschlusseinrichtung und eine an die Pumpenanordnung angeschlossene zweite Anschlusseinrichtung aufweist, wobei die Pumpenanordnung eine Pumpe aufweist, die strömungstechnisch in einem ersten Strömungspfad zwischen der ersten und der zweiten Anschlusseinrichtung verschaltet ist.

Die Beschreibung betrifft weiterhin eine Kanülenanordnung für ein derartiges Herzunterstützungssystem und eine entsprechende Verwendung des Herzunterstützungssystems.

Ein derartiges Herzunterstützungssystem wird in englischer Sprache auch als Ventricular Assist Device (VAD) bezeichnet. Ein solches Herzunterstützungssystem kann sowohl für den linken Ventrikel (LVAD), für den rechten Ventrikel (RVAD) als auch für beide Ventrikel (BVAD) ausgebildet sein.

Häufig steht bei der Anwendung solcher Herzunterstützungssysteme die Blutversorgung des Körpers im Vordergrund, nicht die Entlastung des Herzens zur Ermöglichung einer Therapie der zugrunde liegenden Erkrankung. Üblicherweise führt die Anwendung eines Herzunterstützungssystems zu einer prinzipbedingten Erhöhung der Nachlast des Herzens. Dies erhöht nicht nur die Belastung für das Herz, sondern ist auch von zusätzlichen Schwierigkeiten begleitet, zum Beispiel ein permanentes Verbleiben der Auslass-Herzklappe im geschlossenen Zustand oder gar ein Verwachsen dieser Herzklappe in besagtem geschlossenen Zustand.

Im Zusammenhang mit der vorliegenden Erfindung werden die Gefäße eines Herz-Kreislauf-Systems grundsätzlich als Blutgefäße bezeichnet, unabhängig davon, ob sie Teil des Herzens oder des Kreislaufsystems sind.

Die Druckschrift WO 2014/202051 A1 zeigt ein Herzunterstützungssystem mit einer Systemeinheit, die eine Pumpenanordnung, eine an die Pumpenanordnung angeschlossene ersten Anschlusseinrichtung und eine an die Pumpenanordnung angeschlossene zweite Anschlusseinrichtung aufweist. Die Pumpenanordnung weist dabei eine Pumpe auf, die strömungstechnisch in einem ersten Strömungspfad zwischen der ersten und der zweiten Anschlusseinrichtung verschaltet ist. Das in dieser Druckschrift beschriebene Herzunterstützungssystem ist in der Regel als Herzunterstützungssystem für den linken Ventrikel beschrieben.

Dokument US 5 186 431 A beschreibt ein kardiopulmonales Bypass-System mit mindestens einer Systemeinheit, die eine Pumpenanordnung, eine an die Pumpenanordnung angeschlossene erste Anschlusseinrichtung und eine an die Pumpenanordnung angeschlossene zweite Anschlusseinrichtung auf-weist, wobei die Pumpenanordnung eine erste Pumpe aufweist, die strömungstechnisch in einem ersten Strömungspfad zwischen der ersten und der zweiten Anschlusseinrichtung verschaltet ist, wobei die Pumpenanordnung weiterhin ein Zwischenreservoir und eine zweite Pumpe aufweist, die zusammen mit der ersten Pumpe in einer Serienschaltung im ersten Strömungspfad verschaltet sind, wobei das Zwischenreservoir strömungstechnisch zwischen der ersten Pumpe und der zweiten Pumpe angeordnet ist und wobei die Systemeinheit eine an die Pumpenanordnung angeschlossene dritte Anschlusseinrichtung aufweist, die derart strömungstechnisch mit dem Zwischenreservoir verschaltet ist, dass die zweite Pumpe und das Zwischenreservoir in einer Serienschaltung in einem zweiten Strömungspfad zwischen der zweiten und der dritten Anschlusseinrichtung verschaltet sind. Die Dokumente US 5 270 005 A, US 5 928 179 A und US 2017/112993 A1 zeigen ähnliche Systeme beziehungsweise entsprechende Systemkomponenten.

Es ist die Aufgabe der Erfindung Maßnahmen zur Herzunterstützung bereitzustellen, die bei der Überwindung der eingangs genannten Schwierigkeiten helfen.

Die Lösung der Aufgabe erfolgt erfindungsgemäß durch die Merkmale der unabhängigen Ansprüche. Vorteilhafte Ausgestaltungen der Erfindung sind in den Unteransprüchen angegeben.

Bei dem erfindungsgemäßen Herzunterstützungssystem mit mindestens einer Systemeinheit, die eine Pumpenanordnung, eine an die Pumpenanordnung angeschlossene erste Anschlusseinrichtung und eine an die Pumpenanordnung angeschlossene zweite Anschlusseinrichtung aufweist, wobei die Pumpenanordnung eine erste Pumpe aufweist, die strömungstechnisch in einem ersten Strömungspfad zwischen der ersten und der zweiten Anschlusseinrichtung verschaltet ist, ist vorgesehen, dass die Pumpenanordnung weiterhin ein Zwischenreservoir und eine zweite Pumpe aufweist, die zusammen mit der ersten Pumpe in einer Serienschaltung im ersten Strömungspfad verschaltet sind, wobei das Zwischenreservoir strömungstechnisch zwischen der ersten Pumpe und der zweiten Pumpe angeordnet ist. Das erfindungsgemäße Herzunterstützungssystem ermöglicht es, ein insuffizientes Herz so zu entlasten, dass zum einen der Blutbedarf des Körpers durch das Unterstützungssystem bereits gestellt werden kann, zum anderen eine prinzipbedingte Mehrbelastung des Herzens durch das Herzunterstützungssystem vermieden oder zumindest deutlich unterdrückt wird.

Das Verhalten sowie die Leistung des Herzens des Menschen und anderer Lebewesen wird neben dem Blutbedarf des Körpers im Wesentlichen durch die direkte hydraulische Last beeinflusst. Unterschieden werden die Vorlast und die Nachlast. Bei der Vorlast handelt es sich um die Menge des einströmenden Bluts in die Herzkammern während der Entspannungsphase, welche das Herz bewältigen muss. Die Nachlast resultiert aus dem hydraulischen Widerstand gegen den das Herz während einer Kontraktion ankommen muss. Diese Lasten bestehen jeweils sowohl für die rechte als auch für die linke Herzkammer. Sie sind zudem über das Kreislaufsystem auch untereinander gekoppelt.

Durch die erfindungsgemäßen Maßnahmen ist es möglich, die beiden verschiedenartigen Lasten, welche auf das Herz wirken, nämlich die Vorlast und die Nachlast, unabhängig voneinander einzustellen. Das Herzunterstützungssystem weist dazu eine oder mehrere Systemeinheit(en) zur gezielten Beeinflussung der Druck- und Volumenverläufe im jeweiligen Ventrikel innerhalb des Herzzyklus auf. Die Möglichkeit zu dieser Beeinflussung wird erfindungsgemäß durch die Serienschaltung erste Pumpe-Zwischenreservoir-zweite Pumpe erreicht. Das Zwischenreservoir bildet dabei einen Zwischen- bzw. Pufferspeicher. Durch das Reservoir lässt sich im Zusammenhang mit den Pumpen die Durchflussmenge - zumindest bis zu einem gewissen Grade- temporär von den Druckbedingungen trennen. Mit anderen Worten können die beiden Pumpen, zwischen denen das Zwischenreservoir angeordnet ist, über einen gewissen Zeitraum unterschiedliche Durchflussraten und/oder Druckdifferenzen generieren, was die besagte gezielte Beeinflussung der Druck- und Volumenverläufe ermöglicht. Die Anschlusseinrichtungen sind Anschlusseinrichtungen zum Anschluss an ein jeweiliges Blutgefäß des entsprechenden Herz-Kreislauf-Systems.

Erfindungsgemäß ist weiterhin vorgesehen, dass die Systemeinheit eine an die Pumpenanordnung angeschlossene dritte Anschlusseinrichtung aufweist, die derart strömungstechnisch mit dem Zwischenreservoir verschaltet ist, dass die zweite Pumpe und das Zwischenreservoir in einer Serienschaltung in einem zweiten Strömungspfad zwischen der zweiten und der dritten Anschlusseinrichtung verschaltet sind.

Dabei ist mit Vorteil vorgesehen, dass die Systemeinheit oder bei mehreren Systemeinheiten zumindest eine dieser Systemeinheiten eine Kanülenanordnung für einen derartigen Anschluss der zweiten und der dritten Anschlusseinrichtung an ein Blutgefäß aufweist, dass der Blutstrom direkt durch dieses Blutgefäß an einer Stelle vollständig oder zumindest teilweise gesperrt ist und über den zweiten Strömungspfad mit der Serienschaltung von Zwischenreservoir und zweiter Pumpe umgeleitet wird. Die Kanülenanordnung ist insbesondere als Sperr-Kanülenanordnung (auch Sperrkanüle genannt) ausgebildet, die ein Sperrelement zum vollständigen Sperren des direkten Blutstroms durch das Blutgefäß aufweist.

Erfindungsgemäß ist in dem zweiten Strömungspfad zwischen der zweiten und der dritten Anschlusseinrichtung eine dritte Pumpe so verschaltet, dass sich eine Serienschaltung der zweiten und der dritten Pumpe sowie dem zwischen diesen Pumpen strömungstechnisch verschalteten Zwischenreservoir ergibt.

Gemäß einer bevorzugten Ausführungsform der Erfindung weist die Systemeinheit oder bei mehreren Systemeinheiten zumindest eine dieser Systemeinheiten mindestens eine Kanüle für einen Anschluss der ersten Anschlusseinrichtung und/oder der zweiten Anschlusseinrichtung an ein entsprechendes Blutgefäß auf. Eine derartige Kanüle wird im Weiteren oft auch als herkömmliche Kanüle bezeichnet.

Gemäß einer weiteren bevorzugten Ausführungsform der Erfindung ist vorgesehen, dass die erste Anschlusseinrichtung und die dritte Anschlusseinrichtung saugseitig an die Pumpenanordnung angeschlossen sind und dass die zweite Anschlusseinrichtung druckseitig an die Pumpenanordnung angeschlossen ist.

Gemäß noch einer weiteren bevorzugten Ausführungsform der Erfindung weist das Herzunterstützungssystem zwei Systemeinheiten auf. Die eine Systemeinheit ist dann beispielsweise für den linken Ventrikel und die andere Systemeinheit für den rechten Ventrikel vorgesehen. Das Herzunterstützungssystem ist dann insgesamt ein BVAD.

Dabei ist mit Vorteil vorgesehen, dass das Herzunterstützungssystem eine weitere Pumpe aufweist, die zwischen den Zwischenreservoirs der beiden Systemeinheiten strömungstechnisch zwischengeschaltet ist. Durch diese Maßnahme ergibt sich eine weitere Einstellmöglichkeit.

Gemäß einer weiteren bevorzugten Ausgestaltung der Erfindung weist das Herzunterstützungssystem eine Steuer- und/oder Regeleinrichtung zur Ansteuerung der Pumpen auf.

Gemäß noch einer weiteren bevorzugten Ausgestaltung der Erfindung weist das Herzunterstützungssystem weiterhin Drucksensoren zur Messung des Drucks in Blutgefäßen, Flusssensoren zur Messung der Strömungsgeschwindigkeiten des Bluts in Blutgefäßen und/oder Sensoren zur Messung des Füllvolumens der Herzkammern auf.

Bei der erfindungsgemäßen Kanülenanordnung für ein vorgenanntes Herzunterstützungssystem ist vorgesehen, dass diese für einen derartigen Anschluss der zweiten und der dritten Anschlusseinrichtung an ein Blutgefäß eingerichtet ist, dass der Blutstrom direkt durch dieses Blutgefäß an einer Stelle vollständig oder zumindest teilweise gesperrt ist und über den zweiten Strömungspfad mit der Serienschaltung von Zwischenreservoir und zweiter Pumpe umgeleitet wird. Die Kanülenanordnung ist insbesondere als eine ein Sperrelement zum vollständigen Sperren des direkten Blutstroms durch das Blutgefäß aufweisende Kanülenanordnung ausgebildet.

Bei der erfindungsgemäßen Verwendung ist vorgesehen, dass das vorstehend genannte Herzunterstützungssystem zur therapeutischen Behandlung eines Herzens und/oder zur wissenschaftlichen Untersuchung eines Herzens verwendet wird.

Nachfolgend wird die Erfindung unter Bezugnahme auf die anliegenden Zeichnungen anhand von bevorzugten Ausführungsbeispielen exemplarisch erläutert, wobei die nachfolgend dargestellten Merkmale sowohl jeweils einzeln als auch in Kombination einen Aspekt der Erfindung darstellen können. Es zeigen:
Fig. 1 eine Anordnung mit einem Herz und einem Herzunterstützungssystem gemäß einer ersten bevorzugten Ausführungsform der Erfindung,
Fig. 2 ein Herzunterstützungssystem gemäß einer zweiten bevorzugten Ausführungsform der Erfindung und
Fig. 3 eine Anordnung mit einem Herz und einem anderen Herzunterstützungssystem.

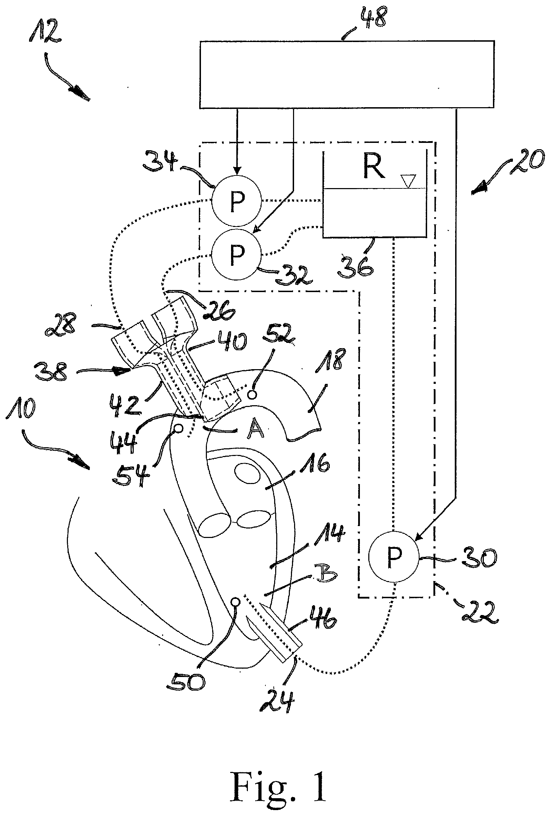

Die Fig. 1 zeigt in schematischer Darstellung ein Herz 10 sowie ein Herzunterstützungssystem 12. Bei der Darstellung des Herzens 10 sind nur wenige Details der linken Herzhälfte sowie deren Anschluss an das Kreislaufsystem, wie beispielsweise die linke Kammer (linker Ventrikel) 14, der linke Vorhof (linkes Atrium) 16 und die Aorta 18, explizit gezeigt.

Das in Fig. 1 gezeigte Herzunterstützungssystem 12 ist ein System für den linken Ventrikel und weist daher nur eine Systemeinheit 20 auf. Dieses Systemeinheit 20 umfasst eine Pumpenanordnung 22, sowie drei an die Pumpenanordnung 22 strömungstechnisch angeschlossene Anschlusseinrichtungen 24, 26, 28. Die Pumpenanordnung 22 weist ihrerseits drei Pumpen 30, 32, 34 sowie ein Zwischenreservoir 36 auf. Die erste dieser Pumpen 30 ist strömungstechnisch in einem ersten Strömungspfad zwischen der ersten und der zweiten der Anschlusseinrichtungen 24, 26 verschaltet, die zweite und die dritte dieser Pumpen 32, 34 sowie das Zwischenreservoir 36 sind in einer Serienschaltung verschaltet strömungstechnisch in einem zwischen der dritten und der zweiten Anschlusseinrichtung 28, 26 verlaufenden zweiten Strömungspfad verschaltet. In dieser Serienschaltung ist das Zwischenreservoir 36 strömungstechnisch zwischen der zweiten und der dritten Pumpe 32, 34 verschaltet. Vom Reservoir 36 aus betrachtet ist die erste Anschlusseinrichtung 24 strömungstechnisch der ersten Pumpe 30, die zweite Anschlusseinrichtung 26 strömungstechnisch der zweiten Pumpe 32 und die dritte Anschlusseinrichtung 28 strömungstechnisch der dritten Pumpe 34 nachgeschaltet.

Die Systemeinheit 20 weist weiterhin eine Kanülenanordnung 38 zweier Kanülen 40, 42 für einen solchen Anschluss der zweiten und der dritten Anschlusseinrichtung 26, 28 an eine Anschlussstelle A in einem Blutgefäß, nämlich die Aorta 18 auf, dass der Blutstrom direkt durch dieses Blutgefäß 18 an einer Stelle bis auf sehr geringe Leckströme gesperrt ist und über den zweiten Strömungspfad mit der Serienschaltung von dritter Pumpe 34, Zwischenreservoir 36 und zweiter Pumpe 32 umgeleitet wird. Die Kanülenanordnung 38 weist zum vollständigen Sperren des direkten Blutstroms durch das Blutgefäß 18 ein von den Wänden der Kanülen 40, 42 gebildetes Sperrelement 44 auf. Schließlich weist die Systemeinheit 20 noch eine Kanüle 46 für einen Anschluss der ersten Anschlusseinrichtung 24 an eine Anschlussstelle B in einem weiteren Blutgefäß, nämlich die Kammer 14 auf.

Das Herzunterstützungssystem 12 umfasst weiterhin ein Regelungssystem mit einer Steuer- und/oder Regeleinrichtung 48 zur Ansteuerung der Pumpen 30, 34, 36 sowie mit Drucksensoren 50, 52, 56 zur Messung des Drucks in den Blutgefäßen 14, 18 auf. Die Drucksensoren 50, 52, 54 sind signaltechnisch mit der Steuer- und/oder Regeleinrichtung 48 verbunden (hier nicht gezeigt). Der erste Drucksensor 50 misst den Druck in der Kammer 14, der zweite Drucksensor 52 misst den Druck in der Aorta 18 hinter dem Sperrelement 44 der Kanülenanordnung 38 der dritte Drucksensor 54 misst den Druck in der Aorta 18 vor dem Sperrelement 44 der Kanülenanordnung 38.

Bei der Systemeinheit 20 des Herzunterstützungssystems 12 handelt es sich um eine elektromechanische Apparatur, die in Kombination mit der Steuer- und/oder Regeleinrichtung 48 zur Interaktion mit dem Herz-Kreislauf-System genutzt wird.

Das Herzunterstützungssystem 12 ist zur Verwendung mit dem lebenden Herz-Kreislauf-System des Menschen oder Tiers bestimmt. Ferner ermöglicht das Herzunterstützungssystem 12 ein systematisches Beforschen sowie Umsetzung einer zielgerichteten, therapeutischen Unterstützung eines erkrankten (insuffizienten) Herzens 10. Das Herzunterstützungssystem 12 ermöglicht dabei ein gezieltes und weitestgehend isoliertes Manipulieren der Herzlasten, insbesondere die beiden verschiedenartigen Lasten (Vor- und Nachlast), für jede Herzkammer unabhängig voneinander einzustellen.

Im Einsatz ermöglicht es das Herzunterstützungssystem 12, ein (erkranktes) Herz 10 so zu be- und entlasten, dass zum einen der Blutbedarf des Körpers durch das System 12 bereit gestellt werden kann, zum anderen die Lastbeaufschlagung des Herzens 10 so gewählt werden kann, dass zum Beispiel (i) in therapeutischer Anwendung die Erholung des erkrankten Herzens 10 optimal gefördert wird. Unter einer derartigen Förderung kann beispielsweise ein gezieltes zeitlich begrenztes Belasten, also ein Training, verstanden werden. Andererseits kann (ii) zu Forschungszwecken über dieses Herzunterstützungssystem 12 eine definierte Lastsituation des Herzens 10 simuliert werden.

Bei herkömmlichen Herzunterstützungssystemen 12 geschieht die Manipulation der Herzlasten ungezielt und unbeabsichtigt gekoppelt, so geht typischerweise mit der Verringerung der Vorlast eine prinzipbedingte Erhöhung der Nachlast einher oder es kommt zu unerwünschten Nebenwirkungen, etwa das Ausbleiben der Öffnung Ausflussherzklappen (Aorten-/Pulmonal-Klappe), sodass die natürliche Funktionsweise des Herzens 10 nicht mehr besteht. Diese Unzulänglichkeiten können durch das Herzunterstützungssystem 12 gelöst werden.

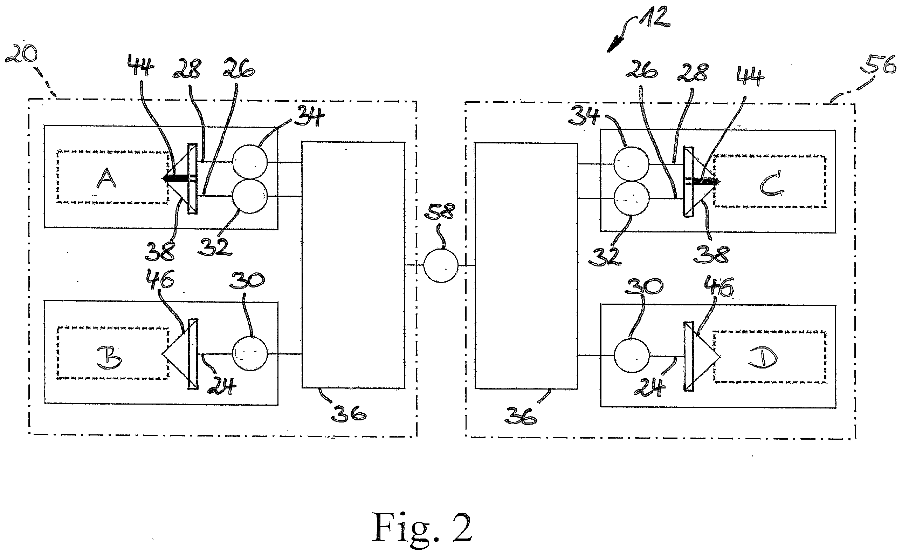

Die Fig. 2 zeigt - ebenfalls in einer schematischen Darstellung - eine weitere Ausführungsform des Herzunterstützungssystems 12, wobei die Regelungskomponenten 48, 50, 52, 54 nicht dargestellt sind. Das in dieser Figur gezeigte Herzunterstützungssystems 12 ist ein Herzunterstützungssystem 12 für beide Ventrikel (BVAD). Es weist daher zwei in ihrer Funktion identisch aufgebaute Systemeinheiten 20, 56 auf. Diese entsprechen in ihrem Aufbau und ihrer Funktionsweise in Wesentlichen der Systemeinheit 20 des in Fig. 1 gezeigten Herzunterstützungssystems 12, sodass im Weiteren vornehmlich auf die Unterschiede eingegangen werden soll.

Um nun die beiden Systemeinheiten 20, 56 gezielt strömungstechnisch miteinander koppeln zu können ist zwischen den beiden Zwischenreservoirs 36 der Systemeinheiten 20, 56 eine weitere Pumpe 58 strömungstechnisch zwischengeschaltet. Auch diese Pumpe 58 wird über die Steuer- und/oder Regeleinrichtung 48 gezielt angesteuert. So ergibt sich ein weiterer Freiheitsgrad bei der Koppelung von Vor- und Nachlast beider Ventrikel.

Die Fig. 2 enthält weiterhin eine Übersicht der vollständigen Konfiguration des Herzunterstützungssystems 12 für beide Ventrikel. Die enthaltenen Komponenten werden im Folgenden genauer beschrieben.

Die in der Fig. 2 benannten Anschluss- beziehungsweise Konnektierungsstellen A, B, C und D sind:
A: die Aorta 18 oder die Pulmonalvene;
B: der linke Ventrikel 14, die Aorta 18, das linke Atrium 16 oder die Pulmonalvene;
C: der rechte Ventrikel, die Pulmonalarterie oder eine der Vena cava; und
D: die Pulmonalarterie oder eine der Vena cava.

Dabei kann es auch mehrere Konnektierungsstellen eines Typs, also zum Beispiel mehrere Konnektierungsstellen vom Typ A, geben.

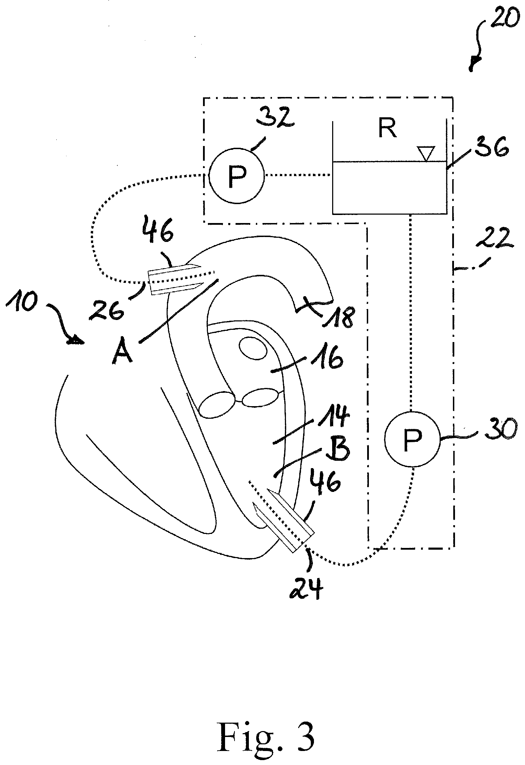

Die Fig. 3 zeigt wieder eine Anordnung mit einem Herz 10 sowie mit einer relativ einfach aufgebauten Systemeinheit 20 eines Herzunterstützungssystems 12. Die Darstellung des Herzens 10 entspricht dabei der Darstellung aus Fig. 1. Auch das hier in Fig. 3 gezeigte Herzunterstützungssystem 12 ist ein System für den linken Ventrikel. Die Systemeinheit 20 umfasst die Pumpenanordnung 22 und nur zwei an die Pumpenanordnung 22 strömungstechnisch angeschlossene Anschlusseinrichtungen 24, 26 (nicht beansprucht). Die Pumpenanordnung 22 weist ihrerseits auch nur zwei Pumpen 30, 32 sowie das Zwischenreservoir 36 auf. Diese Pumpen 30, 32 und das Zwischenreservoir 36 sind in einer Serienschaltung im ersten Strömungspfad zwischen den beiden Anschlusseinrichtungen 24, 26 verschaltet. Dabei ist das Zwischenreservoir 36 strömungstechnisch zwischen der ersten Pumpe 30 und der zweiten Pumpe 34 angeordnet. Die Systemeinheit 20 weist weiterhin zwei Kanülen 46 auf. Über die eine Kanüle 46 erfolgt ein Anschluss der ersten Anschlusseinrichtung 24 an die Anschlussstelle B an der linken Kammer 14 und über die andere Kanüle 46 erfolgt ein Anschluss der zweiten Anschlusseinrichtung 26 an die Anschlussstelle A an der Aorta 18.

Auch ein Herzunterstützungssystem 12 mit einer so simpel aufgebauten Systemeinheit 20 ermöglicht es einerseits, ein insuffizientes Herz 10 derart zu entlasten, dass zum einen der Blutbedarf des Körpers durch das Unterstützungssystem bereits gestellt werden kann, zum anderen dass eine prinzipbedingte Mehrbelastung des Herzens 10 durch das Herzunterstützungssystem 12 vermieden oder zumindest deutlich unterdrückt wird.

Im Weiteren sollen Aufbau, Funktionsweise und Vorteile des Herzunterstützungssystems 12 noch einmal mit anderen Worten beschrieben werden:
Die Gesamtheit des Systems 12 besteht aus einer elektromechanischen Apparatur, die von einer oder mehreren Systemeinheiten 20, 56 gebildet wird, und einer Regelung mit Steuer- und/oder Regeleinrichtung 48 sowie entsprechender Messtechnik (Drucksensoren 50, 52, 54), welche die Ansteuerung der elektromechanischen Apparatur in Interaktion mit dem Herz-Kreislauf-System (als Regelstrecke eines Regelkreises) übernimmt.

Die elektromechanische Apparatur besteht aus der Anordnung 22 hydraulischer Pumpen 30, 32, 34 und Reservoirs 36 für Blut, herkömmlichen Kanülen 46 oder optional neuartiger Sperrkanülen 38 zur Verbindung mit dem Herzkreislauf System an verschiedenen Stellen, sowie entsprechender Verbindungsschläuche.

Hinsichtlich der genauen Konfiguration der Anordnung der Elemente der elektromechanischen Apparatur 20, 56 sind je nach Verwendung der Sperrkanülen 38 sowie der Anzahl der Konnektierungsstellen mit dem Körper verschiedene Ausführungen denkbar. Die Sperrkanülen 38 können in die Blutgefäße 18 unmittelbar hinter den Auswurfklappen des Herzens (Pulmonalarterie oder Aorta 18) oder in den großen Gefäßen vor den Herz-Vorhöfen (Pulmonalvene oder einer der Vena Cava) eingebracht werden. Die Sperrkanülen 38 bezwecken eine hydraulische Entkopplung zwischen Herz 10 und dem sich anschließenden Kreislaufsystem. Die hydraulische Entkopplung durch die Sperrkanülen 38 wird erreicht durch eine Blockierung des Gefäßes, sowie durch Umleiten des vom Herzen 10 ausgeworfenen Bluts aus dem Körper in die Apparatur 20, 56. Gleichzeitig ermöglichen sie die Perfusion des Kreislaufsystems durch Einleitung des aus der Apparatur 20, 56 bereit gestellten Blutstroms in den Körper.

Die konstruktive Ausführung der Sperrkanülen 38 ist sowohl als dauerhaft gesperrt, als auch mit einem Mechanismus zur temporären, reversiblen Sperrung der Kanüle (etwa mittels Ballon oder Klappe) denkbar. Anstatt der neuartigen Sperrkanülen können jedoch auch herkömmliche Kanülen verwendet werden. Herkömmliche Kanülen 46 weisen eine Schlauchverbindungsmöglichkeit auf, die Sperrkanülen 38 weisen zwei Schlauchverbindungsmöglichkeiten auf.

In der Apparatur kommen durch Elektromotoren angetriebene Pumpen 30, 32, 34, 58 zur Förderung des Bluts zur Anwendung.

Charakteristisches Merkmal des Systems 12 ist ein hochdynamischer Betrieb der Pumpen 30, 32, 34, was bedeutet, dass sich die Drehzahlen der Pumpen 30, 32, 34 und dementsprechend der geförderte Blutstrom innerhalb eines Herzzyklus (Herzschlag) signifikant verändern können.

Dementsprechend können geeignet dimensionierte herkömmliche rotatorische Verdrängerpumpen, insbesondere Rotationkolbenpumpen wie Kreiskolbenpumpen, Zahnradpumpen, oder Strömungspumpen wie Kreiselpumpen verwendet werden.

Die Pumpen 30, 32, 34 fördern das Blut in eine oder in beide Richtungen, das heißt sowohl von der Konnektierungsstelle A, B, C, D zum Reservoir 36 als auch umgekehrt.

Kommen Pumpenbauarten zum Einsatz, welche bauartbedingt nur eine Förderrichtung erlauben, zum Beispiel Strömungspumpen, kann durch die Verwendung zwei solcher Pumpen in Verbindung mit Ventilen oder Rückschlagklappen eine Förderung in beide Richtungen erreicht werden. Falls die verwendeten Pumpen 30, 32, 34 den Fluidstrom im Stillstand nicht bauartbedingt sperren, können zusätzliche, beispielsweise elektrisch angesteuerte, Ventile oder Rückschlagklappen verwendet werden. Es können in den Körper implantierbare sowie extrakorporale Pumpen 30, 32, 34, 58 verwendet werden.

Die Reservoirs 36 sammeln das Blut, das von den Pumpen 30, 32, 34 von den Konnektierungsstellen A, B, C, D aus dem Körper hinausbefördert wird. Gleichzeitig stellen die Reservoirs 36 das Volumen (zuvor aus dem Körper hinausbefördertes Blut oder Blutersatzmittel) zur Förderung in den Körper zur Verfügung. Bei den Reservoirs 36 handelt es sich um Behälter aus Glas oder Kunststoff, welche über entsprechende Verbindungsmöglichkeiten mit den Pumpen 30, 32, 34, 58 verfügen. Es können auch flexible Beutel aus Kunststoff genutzt werden. Sofern durch die gewählten Konnektierungsstellen A, B, C, D sauerstoffreiches und sauerstoffarmes Blut gefördert wird, können hierfür zwei getrennte Reservoirs 36 verwendet werden. Es ist jedoch auch ein gemeinsames Reservoir 36 für alle Konnektierungsstellen möglich. Falls zwei Reservoirs 36 verwendet werden, kann durch die Pumpe 58 zwischen den beiden Reservoirs 36 eine Übertragung von sauerstoffreichem Blut in den sauerstoffarmen Kreislauf und umgekehrt erfolgen. Die Reservoirs 36 können wie die Pumpen 30, 32, 34, 58 extrakorporal ausgeführt sein oder in den Körper implantierbar ausgeführt sein.

Zur Verbindung der Komponenten untereinander können Schläuche aus Kunststoff verwendet werden. Die Schläuche können dabei extrakorporal oder intrakorporal sein. Daneben ist jedoch auch eine konstruktive Ausführung der Verbindung denkbar, welche auf Verbindungsschläuche verzichtet, beispielsweise Pumpen 30, 32, 34 mit einer angebauten Kanüle.

Charakteristisches Merkmal des Herzunterstützungssystems 12 ist jedoch, dass mindestens zwei Konnektierungsstellen A, B; C, D auf einer Herzseite und somit mindestens zwei Pumpen 30, 32 erfindungsgemäß mindestens drei Pumpen 30, 32, 34, falls eine vollständig sperrende Sperrkanüle verwendet wird) verwendet werden sowie mindestens ein Reservoir 36.

Die Steuer- und/oder Regeleinrichtung 48 besteht aus einer elektrischen Schaltung und berechnet und erzeugt die Stellsignale für sämtliche verwendeten Pumpen 30, 32, 34. Sie verfügt ferner über Einrichtungen zur Bedienung durch den Benutzer.

Die Berechnung erfolgt unter Berücksichtigung von Messungen, welche durch entsprechende Sensorik und Messtechnik gewonnen werden. Zu den gemessenen Zustandsgrößen können gehören:
Intravasale beziehungsweise ventrikuläre/arterielle Blutdrücke gemessen an den Konnektierungsstellen. Die Druckmessung kann durch herkömmliche invasive Druckmesskatheter oder durch Kanülen mit integriertem Sensor 50, 52, 54 erfolgen.

Das Ventrikelvolumen, gemessen beispielsweise mittels herkömmlichen Conductance-Kathetern oder durch bildgebenden Verfahren wie zum Beispiel Echokardiographie.

Volumenstrom durch die vom Herzen 10 zu und abgehenden Blutgefäße -zum Beispiel mittels Ultraschallsonden gemessen- sowie durch die einzelnen Pumpen 30, 32, 34 der Anordnung, insbesondere dann, wenn kein direkter Zusammenhang zwischen der Pumpendrehzahl und dem Volumenstrom besteht.

Die Blutmenge im Reservoir 36, beispielsweise durch Gewichtsmessung.

Das Herzunterstützungssystem 12 bewirkt einen Eingriff in den ansonsten natürlich vorkommenden Fluss des Blutes in das Herz 10 und aus dem Herzen 10 hinaus. Dieser Eingriff besteht in einer Umverteilung des Bluts.

Die Reduktion der Vorlast wird durch Ausleiten des Bluts aus den Konnektierungsstellen der Gefäße vor dem Herzen 10 sowie aus dem Ventrikel, insbesondere während der Füllungsphase, in das Reservoir 36 erreicht. Eine Erhöhung der Vorlast wird analog durch Einleiten von Volumen (Blut oder Blutersatzflüssigkeit) aus dem Reservoir 36 in die gleichen Konnektierungsstellen erreicht.

Die Reduktion der Nachlast durch die Ausleitung von Blut aus den Konnektierungsstellen der Herzauslassgefäße und aus dem Ventrikel, insbesondere in der Kontraktionsphase, in das Reservoir 36 erreicht. Eine Erhöhung der Nachlast wird durch die Einleitung von Volumen aus dem Reservoir 36 in die gleichen Konnektierungsstellen erreicht.

Charakteristisches Merkmal ist weiterhin, dass diese Umverteilungsflüsse des Blutes nicht konstant sind, sondern innerhalb des Herzzyklus zeitlich veränderlich sind. Ziele sind dabei: (a) Primär: Die präzise Einstellung (Reduktion oder Erhöhung) der Vor- und Nachlast; (b) verallgemeinert: Die gezielte Beeinflussung der Druck- und Volumenverläufe im Ventrikel innerhalb des Herzzyklus und (c) nebenbei: Die gezielte Beeinflussung der Perfusion der Kreisläufe (Drücke und Flüsse).

Der Nutzer des Systems 12 kann die konkreten Ziele und genauen Zielparameter wählen. Die Steuer- und/oder Regeleinrichtung 48 berechnet die für die Einhaltung nötigen Umverteilungsflüsse und Pumpenstellsignale und gibt diese an die Pumpen 30, 32, 34 aus.

### Bezugszeichen

- 10: Herz
- 12: Herzunterstützungssystem
- 14: Kammer (Blutgefäß)
- 16: Vorhof (Blutgefäß)
- 18: Aorta (Blutgefäß)
- 20: Systemeinheit
- 22: Pumpenanordnung
- 24: erste Anschlusseinrichtung
- 26: zweite Anschlusseinrichtung
- 28: dritte Anschlusseinrichtung
- 30: erste Pumpe
- 32: zweite Pumpe
- 34: dritte Pumpe
- 36: Zwischenreservoir
- 38: Kanülenanordnung
- 40: Kanüle
- 42: Kanüle
- 44: Sperrelement
- 46: Kanüle
- 48: Steuer- und/oder Regeleinrichtung
- 50: erster Drucksensor
- 52: zweiter Drucksensor
- 54: dritter Drucksensor
- 56: weitere Systemeinheit
- 58: weitere Pumpe
- A, B, C, D: Anschlussstellen

## Patentansprüche

1. Herzunterstützungssystem (12) mit mindestens einer Systemeinheit (20, 56), die eine Pumpenanordnung (22), eine an die Pumpenanordnung (22) angeschlossene erste Anschlusseinrichtung (24) und eine an die Pumpenanordnung (22) angeschlossene zweite Anschlusseinrichtung (26) aufweist, wobei die Pumpenanordnung (22) eine erste Pumpe (30) aufweist, die strömungstechnisch in einem ersten Strömungspfad zwischen der ersten und der zweiten Anschlusseinrichtung (24, 26) verschaltet ist, wobei die Pumpenanordnung (22) weiterhin ein Zwischenreservoir (36) und eine zweite Pumpe (32) aufweist, die zusammen mit der ersten Pumpe (30) in einer Serienschaltung im ersten Strömungspfad verschaltet sind, wobei das Zwischenreservoir (36) strömungstechnisch zwischen der ersten Pumpe (30) und der zweiten Pumpe (34) angeordnet ist und wobei die Systemeinheit (20, 56) eine an die Pumpenanordnung (22) angeschlossene dritte Anschlusseinrichtung (28) aufweist, die derart strömungstechnisch mit dem Zwischenreservoir (36) verschaltet ist, dass die zweite Pumpe (32) und das Zwischenreservoir (36) in einer Serienschaltung in einem zweiten Strömungspfad zwischen der zweiten und der dritten Anschlusseinrichtung (26, 28) verschaltet sind, **dadurch gekennzeichnet, dass**
in dem zweiten Strömungspfad zwischen der zweiten und der dritten Anschlusseinrichtung (26, 28) eine dritte Pumpe (34) derart verschaltet ist, dass sich eine Serienschaltung der zweiten und der dritten Pumpe (32, 34) sowie dem zwischen diesen Pumpen (32, 34) strömungstechnisch verschalteten Zwischenreservoir (36) ergibt.

2. Herzunterstützungssystem nach Anspruch 1, **dadurch gekennzeichnet, dass** die Systemeinheit (20, 56) eine Kanülenanordnung (38) für einen derartigen Anschluss der zweiten und der dritten Anschlusseinrichtung (26, 28) an ein Blutgefäß (18) aufweist, dass der Blutstrom direkt durch dieses Blutgefäß (18) an einer Stelle vollständig oder zumindest teilweise gesperrt ist und über den zweiten Strömungspfad mit der Serienschaltung von Zwischenreservoir (36) und zweiter Pumpe (32) umgeleitet wird.

3. Herzunterstützungssystem nach einem der Ansprüche 1 bis 2, **dadurch gekennzeichnet, dass** die Systemeinheit (20, 56) mindestens eine Kanüle (46) für einen Anschluss der ersten Anschlusseinrichtung (24) und/oder der zweiten Anschlusseinrichtung (26) an ein entsprechendes Blutgefäß (14, 18) aufweist.

4. Herzunterstützungssystem nach einem der Ansprüche 1 bis 3, **dadurch gekennzeichnet, dass** die erste Anschlusseinrichtung (24) und die dritte Anschlusseinrichtung (28) saugseitig an die Pumpenanordnung (22) angeschlossen sind und dass die zweite Anschlusseinrichtung (26) druckseitig an die Pumpenanordnung (22) angeschlossen ist.

5. Herzunterstützungssystem nach einem der Ansprüche 1 bis 4, **gekennzeichnet durch** zwei Systemeinheiten (20, 56).

6. Herzunterstützungssystem nach Anspruch 5, **gekennzeichnet durch** eine weitere Pumpe (58), die zwischen den Zwischenreservoirs (36) der beiden Systemeinheiten (20, 56) strömungstechnisch zwischengeschaltet ist.

7. Herzunterstützungssystem nach einem der Ansprüche 1 bis 6, **gekennzeichnet durch** eine Steuer- und/oder Regeleinrichtung (48) zur Ansteuerung der Pumpen (30, 32, 34).

8. Herzunterstützungssystem nach einem der Ansprüche 1 bis 7, **gekennzeichnet durch** Drucksensoren (50, 52, 54) zur Messung des Drucks in Blutgefäßen (14, 16, 18) und/oder Flusssensoren zur Messung der Strömungsgeschwindigkeiten des Bluts in Blutgefäßen (14, 16, 18) und/oder Sensoren zur Messung des Füllvolumens der Herzkammern (14).

## Claims

1. Heart support system (12) having at least one system unit (20, 56) which comprises a pump arrangement (22), a first connection means (24) connected to the pump arrangement (22) and a second connection means (26) connected to the pump arrangement (22), wherein the pump arrangement (22) comprises a first pump (30), which is fluidically interconnected in a first flow path between the first and the second connection means (24, 26), wherein the pump arrangement (22) further comprises an intermediate reservoir (36) and a second pump (32), which together with the first pump (30) are interconnected in a series circuit in the first flow path, wherein the intermediate reservoir (36) is arranged fluidically between the first pump (30) and the second pump (34), and wherein the system unit (20, 56) comprises a third connection means (28), which is connected to the pump arrangement (22) and is fluidically connected to the intermediate reservoir (36) in such a way that the second pump (32) and the intermediate reservoir (36) are interconnected in a series circuit in a second flow path between the second and the third connection means (26, 28), **characterized in that** a third pump (34) is interconnected in the second flow path between the second and the third connection means (26, 28) in such a way that a series circuit of the second and the third pumps (32, 34) and the intermediate reservoir (36) fluidically interconnected between these pumps (32, 34) is obtained.

2. Heart support system according to Claim 1, **characterized in that** the system unit (20, 56) comprises a cannula arrangement (38) for such a connection of the second and the third connection means (26, 28) to a blood vessel (18) that the blood flow directly through this blood vessel (18) is completely or at least partially blocked at one location and is diverted via the second flow path with the series circuit of intermediate reservoir (36) and second pump (32).

3. Heart support system according to either of Claims 1 and 2, **characterized in that** the system unit (20, 56) comprises at least one cannula (46) for a connection of the first connection means (24) and/or the second connection means (26) to a corresponding blood vessel (14, 18).

4. Heart support system according to one of Claims 1 to 3, **characterized in that** the first connection means (24) and the third connection means (28) are connected at the suction side to the pump arrangement (22), and **in that** the second connection means (26) is connected at the pressure side to the pump arrangement (22).

5. Heart support system according to one of Claims 1 to 4, **characterized by** two system units (20, 56).

6. Heart support system according to Claim 5, **characterized by** a further pump (58), which is fluidically interconnected between the intermediate reservoirs (36) of the two system units (20, 56).

7. Heart support system according to one of Claims 1 to 6, **characterized by** an open-loop and/or closed-loop control means (48) for controlling the pumps (30, 32, 34) .

8. Heart support system according to one of Claims 1 to 7, **characterized by** pressure sensors (50, 52, 54) for measuring the pressure in blood vessels (14, 16, 18), and/or flow sensors for measuring the flow velocities of the blood in blood vessels (14, 16, 18), and/or sensors for measuring the filling volume of the ventricles (14).

## Revendications

1. Système d'assistance cardiaque (12) avec au moins une unité de système (20, 56) qui présente un agencement de pompe (22), un premier appareil de raccordement (24) raccordé à l'agencement de pompe (22) et un deuxième appareil de raccordement (26) raccordé à l'agencement de pompe (22), l'agencement de pompe (22) présentant une première pompe (30) qui est connectée en termes d'écoulement dans un premier trajet d'écoulement entre le premier et le deuxième appareil de raccordement (24, 56), 26), l'agencement de pompe (22) présentant en outre un réservoir intermédiaire (36) et une deuxième pompe (32) qui sont connectés en série conjointement avec la première pompe (30) dans le premier trajet d'écoulement, le réservoir intermédiaire (36) étant agencé en termes d'écoulement entre la première pompe (30) et la deuxième pompe (34), et l'unité de système (20, 56) présentant un troisième appareil de raccordement (28) raccordé à l'agencement de pompe (22), qui est connecté en termes d'écoulement au réservoir intermédiaire (36) de telle sorte que la deuxième pompe (32) et le réservoir intermédiaire (36) sont connectés en série dans un deuxième trajet d'écoulement entre le deuxième et le troisième appareil de raccordement (26, 28), **caractérisé en ce que**
une troisième pompe (34) est connectée dans le deuxième trajet d'écoulement entre le deuxième et le troisième appareil de raccordement (26, 28) de telle sorte qu'il en résulte une connexion en série de la deuxième et de la troisième pompe (32, 34) ainsi que du réservoir intermédiaire (36) connecté en termes d'écoulement entre ces pompes (32, 34).

2. Système d'assistance cardiaque selon la revendication 1, **caractérisé en ce que** l'unité de système (20, 56) présente un agencement de canule (38) pour un raccordement du deuxième et du troisième appareil de raccordement (26, 28) à un vaisseau sanguin (18) tel que le flux sanguin est complètement ou au moins partiellement bloqué à un emplacement directement à travers ce vaisseau sanguin (18) et est redirigé par l'intermédiaire du deuxième trajet d'écoulement avec la connexion en série du réservoir intermédiaire (36) et de la deuxième pompe (32).

3. Système d'assistance cardiaque selon l'une quelconque des revendications 1 à 2, **caractérisé en ce que** l'unité de système (20, 56) présente au moins une canule (46) pour un raccordement du premier appareil de raccordement (24) et/ou du deuxième appareil de raccordement (26) à un vaisseau sanguin correspondant (14, 18) .

4. Système d'assistance cardiaque selon l'une quelconque des revendications 1 à 3, **caractérisé en ce que** le premier appareil de raccordement (24) et le troisième appareil de raccordement (28) sont raccordés côté aspiration à l'agencement de pompe (22) et **en ce que** le deuxième appareil de raccordement (26) est raccordé côté refoulement à l'agencement de pompe (22).

5. Système d'assistance cardiaque selon l'une quelconque des revendications 1 à 4, **caractérisé par** deux unités de système (20, 56).

6. Système d'assistance cardiaque selon la revendication 5, **caractérisé par** une autre pompe (58) qui est interconnectée en termes d'écoulement entre les réservoirs intermédiaires (36) des deux unités de système (20, 56).

7. Système d'assistance cardiaque selon l'une quelconque des revendications 1 à 6, **caractérisé par** un appareil de commande et/ou de régulation (48) pour la commande des pompes (30, 32, 34).

8. Système d'assistance cardiaque selon l'une quelconque des revendications 1 à 7, **caractérisé par** des capteurs de pression (50, 52, 54) pour mesurer la pression dans des vaisseaux sanguins (14, 16, 18) et/ou des capteurs de débit pour mesurer les vitesses d'écoulement du sang dans des vaisseaux sanguins (14, 16, 18) et/ou des capteurs pour mesurer le volume de remplissage des cavités cardiaques (14).
